# EUROPEAN PATENT APPLICATION

(11) **EP 4 434 563 A1**
(43) Date of publication of application: **25.09.2024**
(21) Application number: 23163686.1
(22) Date of filing: 23.03.2023
(51) Int. Cl.: A61M 11/04, A61M 15/06, A61M 16/08

(54) **A HANDHELD DEVICE FOR VAPORIZING LIQUID TO BE INHALED BY A USER AND A METHOD FOR MANUFACTURING A HANDHELD DEVICE**

(71) Applicant: EQOY International Group AG, 6300 Zug (CH)
(72) Inventor: FREIENMUTH, Samuel, 8953 Dietikon (CH); PRENTER, Torsten, 8706 Meilen (CH)
(74) Representative: 2SPL Patentanwälte PartG mbB

(57) **Abstract**

A handheld device for vaporizing liquid to be inhaled by a user comprises a mouthpiece, a heating structure for vaporizing liquid and a liquid reservoir configured to provide a liquid to the heating structure. The liquid reservoir is configured to bind the liquid before the liquid is provided to the heating structure so that less than 20% of all the liquid within the handheld device is free.

## Description

### Field

The disclosure relates to concepts for vaporizing liquid, which is afterwards inhaled by a user.

### Background

A large variety of electronic cigarettes and inhalation devices are known. Often a liquid is heated to high temperatures to produce vapor that can be inhaled by the user. Unhealthy components can result from excessive heating.

### Summary

Examples relate to a handheld device for vaporizing liquid to be inhaled by a user. The handheld device comprises a mouthpiece and a heating structure comprising a ceramic tube. Further, the handheld device comprises a first electrically conductive structure connected to a first electrode of the heating structure being in contact with the ceramic tube. Additionally, the handheld device comprises a second electrically conductive structure connected to a second electrode of the heating structure being in contact with the ceramic tube. At least a part of the ceramic tube is located between the first electrode and the second electrode.

Some examples relate to a handheld device for vaporizing liquid to be inhaled by a user. The handheld device comprises a mouthpiece, a heating structure for vaporizing liquid and a liquid reservoir configured to provide a liquid to the heating structure. The liquid reservoir is configured to bind the liquid before the liquid is provided to the heating structure so that less than 20% of all the liquid within the handheld device is free.

Further examples relate to a method for manufacturing of a handheld device for vaporizing liquid to be inhaled by a user. The method comprises filling a liquid into a housing of a handheld device so that a liquid reservoir absorbs the liquid so that less than 20% of all the liquid within the handheld device is free after sealing the handheld device. Further, the method comprises sealing the handheld device so that the liquid is not refillable.

### Brief description of the Figures

Some examples of apparatuses and/or methods will be described in the following by way of example only, and with reference to the accompanying figures, in which
Fig. 1 shows a schematic cross section of a handheld device for vaporizing liquid;
Fig. 2a shows a schematic longitudinal section of a ceramic tube of a heating structure;
Fig. 2b shows a schematic cross section of the ceramic tube of Fig. 2a;
Fig. 3 shows a schematic cross section of a handheld device for vaporizing liquid indicating an air flow;
Fig. 4 shows a schematic cross section of a handheld device for vaporizing liquid; and
Fig. 5 shows a flow chart of a method for manufacturing of a handheld device.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples. Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e. only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not xcludee the presence or addition of one or more other features, integers, steps, operations, processes, elements, components and/or a group thereof.

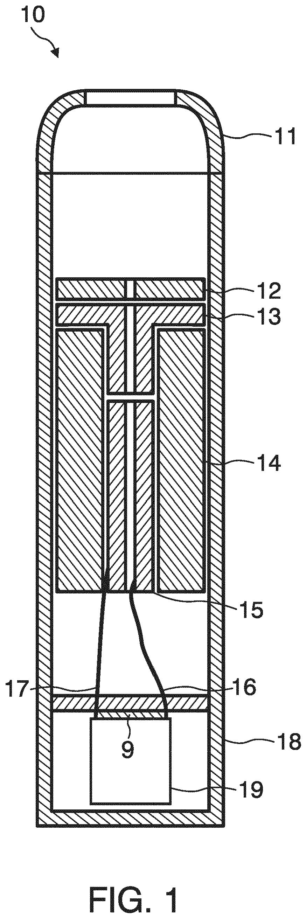

Fig. 1 shows a handheld device 10 (e.g. for vaporizing liquid to be inhaled by a user). The handheld device 10 comprises a mouthpiece 11 and a heating structure 15 comprising a ceramic tube. Further, the handheld device 10 comprises a first electrically conductive structure 16 connected to a first electrode of the heating structure 15 being in contact with the ceramic tube. Additionally, the handheld device 10 comprises a second electrically conductive structure 17 connected to a second electrode of the heating structure 15 being in contact with the ceramic tube. At least a part of the ceramic tube is located between the first electrode and the second electrode.

By using a ceramic structure for vaporizing a liquid, the temperature can be easily controlled and vapor can be produced at low temperatures. In this way, undesired or unhealthy components in the vapor or smoke can be reduced or avoided. It may be possible to provide clean vapor or smoke.

The temperature, which the ceramic tube reaches during operation, may depend on the electrical resistance of the ceramic tube between the first electrode and the second electrode and the voltage applied between the first electrode and the second electrode. The electrical resistance of the ceramic tube between the first electrode and the second electrode may depend on the material of the ceramic tube and the distance between the first electrode and the second electrode.

For example, a battery (e.g. primary or secondary battery) may provide a supply voltage and the heating structure 15 may comprise an electrical resistance so that the ceramic tube is heated to a temperature of at least 100 °C (or at least 90°C, at least 110°C or at least 120°C) and/or at most 190 °C (or at most 170°C, at most 150°C or at most 210°C), when a current flow through the ceramic tube is activated for at least 0.5 s (or at least 1s or at least 1.5 s) and/or at most 4 s (or at most 3 s or at most 2 s). A user may take a drag on the handheld device 10 for approximately 1-3 s per inhalation cycle. During that time, the ceramic tube may be heated to more than 100°C, but less than 190°C.

For example, the ceramic tube may comprise, may be made of or may be essentially composed of (e.g. non-porous or porous) aluminum oxide (e.g. Al2O3), zirconium oxide (e.g. ZrO2) or other ceramic material. For example, the ceramic tube may be an aluminum oxide tube. For example, the ceramic tube may comprise, may be made of or may be essentially composed of porous ceramic material. The porous ceramic material may have a porosity of more than 20% (or more than 30% or more than 50%). By using a porous material for the ceramic tube, the liquid to be vaporized may flow into the pores of the porous material and may be easily vaporized and exit the ceramic tube through the central opening. For example, the ceramic tube may be a porous aluminum oxide tube.

For example, an electrical resistance between the first electrode and the second electrode through at least a part of the ceramic tube (e.g. the part of the ceramic tube, which is located between the first electrode and the second electrode) is at least 0.20 ohm (or at least 0.3 ohm, at least 0.5 ohm, at least 1 ohm or at least 1.5 ohm) and/or at most 3.3 ohm (or at most 3 ohm or at most 2.5 ohm), for example, 2.2 ohm. For example, the distance between the first electrode and the second electrode may be selected so that the part of the ceramic tube, which is located between the first electrode and the second electrode, has the desired electrical resistance.

For example, the ceramic tube may comprise a length of at least 1 cm (or at least 1.5 cm or at least 2 cm) and/or at most 6 cm (or at most 3 cm or at most 6 cm). The ceramic tube may comprise a central opening with a diameter of at least 3 mm (or at least 4 mm or at least 5 mm) and/or at most 8 mm (or at most 7 mm or at most 6 mm). The ceramic tube may comprise an outer diameter of at least 1.1 mm (or at least 2.5 mm or at least 4 mm) and/or at most 8.2 mm (or at most 7 mm or at least 6 mm).

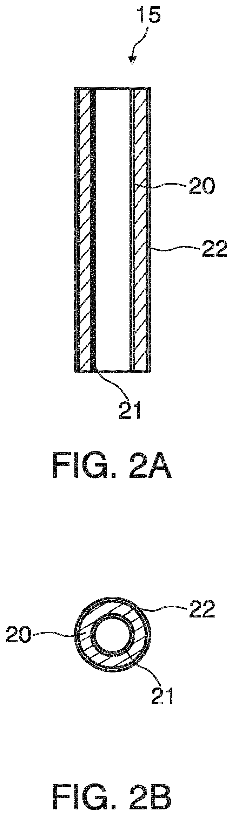

The ceramic tube of the heating structure 15 may be a hollow ceramic structure with an opening at both ends. A cross section of the ceramic tube of the heating structure 15 may comprise a circular or a rectangular geometry or any other tube-like geometry. Fig. 2a and 2b show an example of a of a ceramic tube with a circular geometry. The first electrode 21 of the heating structure 15 may be in contact with an inner surface of the ceramic tube 20 and may extend over the complete inner surface or only a part of the inner surface of the ceramic tube 20. The second electrode 22 of the heating structure 15 may be in contact with an outer surface of the ceramic tube 20 and may extend over the complete outer surface or only a part of the outer surface of the ceramic tube 20. In this example, the electrical resistance between the first electrode 21 and the second electrode 22 may be set by the thickness of the ceramic tube. Alternatively, the first electrode 21 and the second electrode 22 may both be located at the inner surface or at the outer surface of the ceramic tube 20. In this example, the electrical resistance between the first electrode and the second electrode may be set by the distance between the first electrode 21 and the second electrode 22 on the inner surface or the outer surface of the ceramic tube 20.

Instead of a ceramic tube, the heating structure 15 may comprise a ceramic structure with another geometry (e.g. a ceramic rod, a ceramic ring or a ceramic cube) with at least a part of the ceramic structure being located between a first electrode and a second electrode. For example, the first electrode and the second electrode may be located at opposite ends of a ceramic rod, at different areas of a ceramic ring or on opposite sides of a ceramic cube. The ceramic structure may be made of or may be essentially composed of one of the materials and/or may have dimensions mentioned in connection with the ceramic tube. The heating structure 15 may comprise an electrical resistance between the first electrode and the second electrode as mentioned above.

The heating structure 15 may be configured to heat the ceramic tube to a temperature of at least 100 °C (or at least 90°C, at least 110°C or at least 120°C) and/or at most 190 °C (or at most 170°C, at most 150°C or at most 210°C). For example, the ceramic tube and the positions of the electrodes may be selected so that the heating structure 15 enables temperatures in the mentioned range. For example, the heating structure 15 may be configured to provide a power of at least 4 Wat (or at least 6 Watt or at least 8 Watt) and/or at most 18 Watt (or at most 15 Watt or at most 12 Watt).

The first electrically conductive structure 16 and the second electrically conductive structure 17 may be or may comprise wires (e.g. copper wires) or electrically conductive traces (e.g. formed on a surface).

The first electrically conductive structure 16 may electrically connect the first electrode to a battery or an activation apparatus of the handheld device 10. The second electrically conductive structure 17 may electrically connect the second electrode to a battery or an activation apparatus of the handheld device 10.

The first electrode and/or the second electrode may be attached to the ceramic tube or may be deposited on the ceramic tube. The first electrode and/or the second electrode may comprise or may be made of copper or any other electrically conductive material.

The handheld device 10 may comprise an activation apparatus 9 configured to activate a current flow from the first electrically conductive structure 16 to the second electrically conductive structure 17 through at least a part of the ceramic tube, when a user sucks (e.g. takes a drag) at the mouthpiece 11. For example, the activation apparatus 9 may comprise a pressure sensor. The pressure sensor may be configured to detect a pressure and/or a change of pressure within the handheld device 10. The activation apparatus 9 may be configured to activate the current flow, if the pressure sensor detects a pressure equal to or below a pressure threshold and/or a change of pressure exceeding a pressure change threshold. The activation apparatus 9 may be configured to deactivate the current flow, if the pressure sensor detects a pressure equal to or above a pressure threshold and/or a change of pressure decreases below a pressure change threshold. Alternatively, the activation apparatus 9 configured to activate a current flow from the first electrically conductive structure 16 to the second electrically conductive structure 17 through at least a part of the ceramic tube, when a user activates the handheld device 10 (e.g. by pushing a button). The activation apparatus 9 may comprise hardware (e.g. processor circuitry, for example, a microcontroller or ASIC or FGPA) and/or software to provide the described functionality.

The handheld device 10 may further comprise a non-removable or removeable battery (e.g. primary or secondary battery). The battery 19 may be coupled to the first electrically conductive structure 16 and the second electrically conductive structure 17. The battery 19 may be directly connected the first electrically conductive structure 16 or coupled through the activation apparatus 9. The battery 19 may be directly connected the second electrically conductive structure 17 or coupled through the activation apparatus 9. The battery 19 may be connected to the activation apparatus 9. The battery 19 may provide a supply voltage for the activation apparatus 9 and/or the heating structure 15. The battery may provide a supply voltage of at least 1V (or at least 2V or at least 3V) and/or at most 8V (or at most 6V or at most 5V). The battery 19 may provide at least 200 mAh and/or at most 4000 mAh, for example 450 mAh. The battery 19 (e.g. sensor-controlled battery) may be air-draw activatable to power the handheld device 10. The battery 19 may be located in a battery compartment within the housing 18, which is sealed from a cavity extending to the mouthpiece 11. The battery compartment may be located at a bottom end of the handheld device 10. The battery compartment may comprise one or more venting holes.

The mouthpiece 11 may be located at a top end of the handheld device 10. The mouthpiece 11 may comprise or may be made of polyoxymethylene with an antibacterial coating (e.g. which keeps bacteria low) or any other suitable material and/or coating.

The handheld device 10 may further comprise a liquid reservoir 14 configured to provide a liquid to the heating structure 15. The liquid reservoir 14 may comprise a container and a dispenser for providing liquid to the heating structure 15 or the liquid reservoir 14 may comprise a liquid absorbing material in contact or in proximity with the heating structure 15. The liquid may be a liquid comprising water, flavorings, nicotine (e.g. nicotine salt), glycerin (e.g. vegetable glycerin), propylene glycol and/or other ingredients. The liquid may be any liquid which can be vaporized in the temperature range mentioned above and which may provide the user with desired flavors, nicotine and/or medical substances. The user may be a smoker or a patient.

The handheld device 10 may further comprise a sealing structure 13 located between the mouthpiece 11 and the ceramic tube. The sealing structure 13 may be configured to partially seal a first cavity located between the mouthpiece 11 and the sealing structure 13 from a second cavity that houses the ceramic tube within a housing 18 of the handheld device 10. For example, the sealing structure 13 may seal the first cavity from the second cavity except for a central opening or except for a central opening and one or more circumferential openings located between the sealing structure 13 and the housing 18 of the handheld device 10. For example, the central opening of the sealing structure 13 may be aligned with the central opening of the ceramic tube. The central opening of the sealing structure 13 and/or the circumferential openings located between the sealing structure 13 and the housing of the handheld device 10 may enable vapor to move from the second cavity to the first cavity. The sealing structure 13 may comprise or may be made of silicone (e.g. NSF-51) or any other heat resistant material and/or food grade material.

The handheld device 10 may further comprise an absorbent structure 12 located between the mouthpiece 11 and the sealing structure 12. The absorbent structure 12 may be configured to absorb liquid which may exit the second cavity through the central opening of the sealing structure 13 and/or the circumferential openings between the sealing structure 13 and the housing of the handheld device 10 or which condenses in the first cavity. In this way, it can be avoided or reduced that liquid reaches the user through the mouthpiece 11. The absorbent structure 12 may comprise or may be made of cotton or another absorbing material. The cotton may absorb liquid (e.g. water) to avoid getting liquid into the mouth.

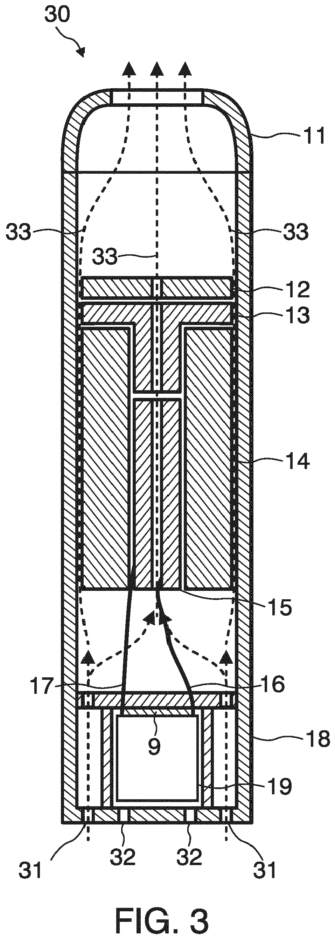

The handheld device 10 may also be called inhalation device, inhaler, electronic inhalation device, clean inhaler, clean inhalation device or electronic cigarette. For example, the handheld device 10 has a size and weight so that it can easily be carried by a user in one hand. For example, the handheld device 10 may comprise a maximal length of at most 12 cm (or at most 15 cm or at most 10 cm). The handheld device 10 may comprise a weight of at most 150 g (or at most 120 g or at most 100 g). The housing 18 of the handheld device 10 may comprise or may be made of aluminum or any other lightweight, but stable material. For example, the housing 18 may comprise a leak-proof tube-shaped main part (e.g. coated aluminum tube), an open top for attaching the mouthpiece 11 and a bottom with one or more air openings and/or one or more venting holes (e.g. shown in Fig. 3). The bottom (e.g. a cap) of the housing 18 may be removable for exchange of the battery 9. Alternatively, the bottom may be non-removable for a single-use handheld device 10.

The handheld device 10 may enable a non-toxic and/or low-emission inhalation of flavorings with or without nicotine or a nicotine replacement therapy.

Fig. 3 shows an example of an air flow 33 within a handheld device 10. The handheld device 30 may be implemented similar to the handheld device described in connection with Fig. 1.

The handheld device 30 may comprise one or more openings 31 at a bottom of the handheld device 30. In addition, one or more venting holes 32 for a battery compartment may be located at the bottom of the handheld device 30.

If the user sucks at the mouthpiece 11, air flows from the one or more air openings 31 through a cavity containing the ceramic tube to the mouthpiece 11. In this way, vapor generated by heating the ceramic tube can be carried to the mouthpiece and can be inhaled by the user.

More details and aspects are mentioned in connection with the examples described above or below. The handheld device 30 may comprise one or more optional additional features corresponding to one or more aspects mentioned in connection with the proposed concept or one or more examples described above (e.g. Fig. 1-2b) or below.

Some examples relate to a method for vaporizing liquid to be inhaled by a user. The method comprises heating a ceramic structure (e.g. a ceramic tube) of a heating structure of a handheld device so that liquid is vaporized by activating a current flow from a first electrically conductive structure connected to a first electrode of the heating structure being in contact with the ceramic tube to a second electrically conductive structure connected to a second electrode of the heating structure being in contact with the ceramic tube. At least a part of the ceramic tube is located between the first electrode and the second electrode. More details and aspects are mentioned in connection with the examples described above or below. The method may comprise one or more optional additional features corresponding to one or more aspects mentioned in connection with the proposed concept or one or more examples described above (e.g. Fig. 1-3) or below.

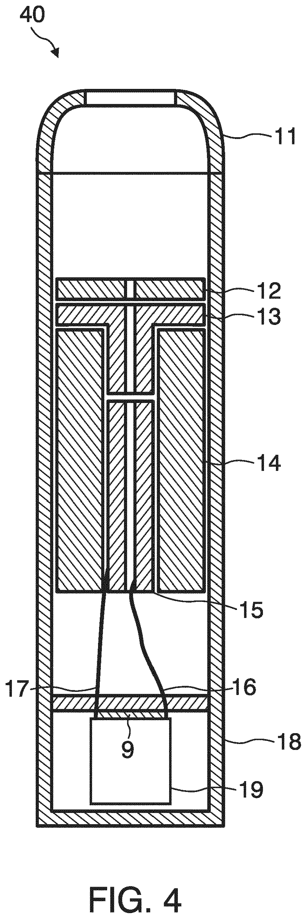

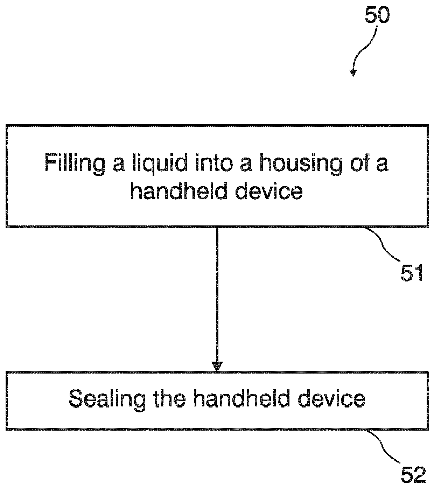

Fig. 4 shows a handheld device 40 (e.g. for vaporizing liquid to be inhaled by a user). The handheld device 40 comprises a mouthpiece 11 and a heating structure 15 for vaporizing liquid. Further, the handheld device 10 comprises a liquid reservoir 14 configured to provide a liquid to the heating structure 15. The liquid reservoir 14 is configured to bind, absorb, soak up and/or store the liquid before the liquid is provided to the heating structure 15 so that less than 20% (or less than 15%, less than 10% or less than 5%) of all the liquid within the handheld device 10 is free.

By avoiding free liquid in the handheld device, it may be possible to avoid spilling significant amounts of the liquid if the housing of the handheld device is damaged.

It may be understood that the liquid is only bound, absorbed, soaked up and/or stored by the liquid reservoir 14 to a degree that it can move within the liquid reservoir 14 and can be provided to the heating structure 15 (e.g. due to capillary action), but cannot flow freely within the handheld device 10. The liquid reservoir 14 may be configured to enable liquid to move inside the liquid reservoir towards the heating structure 15. The liquid reservoir 14 may be configured to bind the liquid before the liquid is provided to the heating structure 15 so that less than 5% (or less than 20%, less than 15% or less than 10%) of the all the liquid within the handheld device is free (e.g. can flow freely).

The liquid reservoir 14 may comprise or may consist of material, which is able to bind, absorb, soak up and/or store liquids. For example, the liquid reservoir 14 may comprise or may consist of a liquid absorbing material. The liquid reservoir 14 may comprise or may consist of cotton (e.g. an unbleached, natural cotton or Japanese filtered unbleached natural Muji cotton), hemp wool, silicate cord or a sponge.

The liquid reservoir 14 may be in contact with the heating structure 15 or in close proximity to the heating structure 15. For example, a gap between the liquid reservoir 14 and the heating structure 15 may be at most 2 mm (or at most 1 mm or at most 500 µm). In this way, a part of the liquid stored in the liquid reservoir 14, which is close to the heating structure 15 may be vaporized and/or liquid may be provided to the heating structure 15 and may be vaporized at a surface or in the heating structure 15, when the heating structure 15 is heated. For example, if the heating structure 15 comprises a ceramic structure, liquid may move from the liquid reservoir 14 into the ceramic structure and the liquid in the ceramic structure may be vaporized, when the ceramic structure is heated.

The liquid reservoir 14 may enable a continuous supply of liquid to the heating structure 15 since the liquid may be able to move within the liquid reservoir 14 due to capillary action. As soon as liquid has moved from the liquid reservoir 14 to the heating structure 15 or is vaporized at an interface between the liquid reservoir 14 and the heating structure 15, more liquid will move from within the liquid reservoir 14 to the heating structure 15. It may be possible to enable a continuous supply of liquid to the heating structure 15 independent from a holding position of the handheld device 10. Even if the user may hold the handheld device 10 upside down, the liquid supply may be ensured.

The liquid reservoir 14 may encircle the heating structure 15 or the heating structure 15 may encircle the liquid reservoir 14. For example, the liquid reservoir 14 may be ring-shaped or tube-shaped. The heating structure 15 may be located in an opening of the liquid reservoir 14. Alternatively, the heating structure 15 may be ring-shaped or tube-shaped. The liquid reservoir 14 may be located in an opening of the heating structure 15.

The liquid reservoir 14 may be configured to store at least 1 ml (or at least 5 ml or at least 10 ml) of liquid and/or at most 20 ml (or at most 30 ml or at most 15 ml) of liquid. For example, at most 20 ml (or at most 30 ml or at most 15 ml) and/or at least 1 ml (or at least 5 ml or at least 10 ml) of liquid is in the handheld device 40. The handheld device 40 may get too large, if more than 30 ml of liquid is in the handheld device 40. The usage time may get too low, if less than 1 ml of liquid is in the handheld device 40.

The liquid may comprise nicotine or may be nicotine-free. The liquid may comprise less than 70 % (or less than 50%, less than 30% or less than 20%) of water or may be waterless.

The heating structure 15 may be any structure (e.g. a resistor) heatable by a flow of electric current. For example, the heating structure 15 may comprise a ceramic structure (e.g. a ceramic tube) as described in connection with one or more of the examples of Fig. 1-3. The liquid reservoir 14 may be in contact with the ceramic tube.

The handheld device 40 may be configured so that the liquid is not refillable. For example, the liquid reservoir 14 may be located in a housing of the handheld device 40 without the ability to be replaced or filled with new liquid. For example, the liquid reservoir 14 may be located in a sealed space of the handheld device 40, which can only be opened by destroying the handheld device 40. For example, the handheld device 40 may be a disposable device, a single-use device or a one-way device.

More details and aspects are mentioned in connection with the examples described above or below. The handheld device 40 may comprise one or more optional additional features corresponding to one or more aspects mentioned in connection with the proposed concept or one or more examples described above (e.g. Fig. 1-3) or below.

Fig. 5 shows a flow chart of a method for manufacturing of a handheld device for vaporizing liquid to be inhaled by a user. The method 50 comprises filling 51 a liquid to be vaporized into a housing (or enclosure) of a handheld device so that a liquid reservoir absorbs the liquid so that less than 20% of all the liquid within the handheld device is free after sealing the handheld device. Further, the method 50 comprises sealing 52 the handheld device so that the liquid is not refillable.

The liquid may be filled into a chamber of the housing, which houses the liquid reservoir so that the liquid reservoir can absorb, bind, soak up and/or store the liquid.

The handheld device may be sealed by sealing an opening, which was used for filling the liquid into the housing. For example, the handheld device may be sealed by attaching a mouthpiece to the housing. The handheld device may be sealed in a way that it can only be opened by destroying the handheld device.

The handheld device may comprise a mouthpiece attached to the housing. Further, the handheld device may comprise a heating structure for vaporizing the liquid. The heating structure may be located in the chamber housing the liquid reservoir.

The method may further comprise placing the liquid reservoir and/or the heating structure in the housing before filling the liquid into the housing.

More details and aspects are mentioned in connection with the examples described above or below. The method 50 may comprise one or more optional additional features corresponding to one or more aspects mentioned in connection with the proposed concept or one or more examples described above (e.g. Fig. 1-4) or below.

The aspects and features described in relation to a particular one of the previous examples may also be combined with one or more of the further examples to replace an identical or similar feature of that further example or to additionally introduce the features into the further example.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, -functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

## Claims

1. A handheld device (10) for vaporizing liquid to be inhaled by a user, comprising:
a mouthpiece (11);
a heating structure (15) for vaporizing liquid; and
a liquid reservoir (14) configured to provide a liquid to the heating structure (15), wherein the liquid reservoir (14) is configured to bind the liquid before the liquid is provided to the heating structure (15) so that less than 20% of all the liquid within the handheld device (10) is free.

2. The handheld device of claim 1, wherein the liquid reservoir (14) comprises a liquid absorbing material.

3. The handheld device of one of the previous claims, wherein the liquid reservoir (14) is a cotton, hemp wool, silicate cord or a sponge.

4. The handheld device of claim 3, wherein the cotton is an unbleached, natural cotton.

5. The handheld device of one of the previous claims, wherein the liquid reservoir (14) is in contact with the heating structure.

6. The handheld device of one of the previous claims, wherein the liquid reservoir (14) is configured to enable liquid to move inside the liquid reservoir (14) towards the heating structure (15).

7. The handheld device of one of the previous claims, wherein the liquid reservoir (14) encircles the heating structure (15).

8. The handheld device of one of the previous claims, wherein the handheld device (10) is configured so that the liquid is not refillable.

9. The handheld device of one of the previous claims, wherein at most 20 ml of liquid is in the handheld device (10).

10. The handheld device of one of the previous claims, wherein the liquid reservoir (14) is configured to bind the liquid before the liquid is provided to the heating structure (15) so that less than 5% of the all the liquid within the handheld device (10) is free.

11. The handheld device of one of the previous claims, wherein the heating structure (15) comprises a ceramic tube, wherein the liquid reservoir (14) is in contact with the ceramic tube.

12. The handheld device of one of the previous claims, wherein the liquid comprises less than 70 % of water.

13. The handheld device of one of the previous claims, wherein the liquid comprises nicotine.

14. The handheld device of one of the previous claims, wherein the liquid reservoir (14) is configured to store at least 1 ml of liquid and at most 20 ml of liquid.

15. A method (50) for manufacturing a handheld device for vaporizing liquid to be inhaled by a user, the method comprising:
Filling (51) a liquid to be vaporized into a housing of a handheld device so that a liquid reservoir absorbs the liquid so that less than 20% of all the liquid within the handheld device is free after sealing the handheld device; and
Sealing (52) the handheld device so that the liquid is not refillable.
